# EUROPÄISCHE PATENTSCHRIFT

(11) **EP 1 880 698 B1**
(45) Veröffentlichungstag und Bekanntmachung des Hinweises auf die Patenterteilung: **30.09.2009**
(21) Anmeldenummer: 06405317.6
(22) Anmeldetag: 21.07.2006
(51) Int. Cl.: A61F 9/008, A61F 9/01

(54) **Ophthalmologische Vorrichtung**
Ophthalmological device
Dispositif ophthalmologique

(43) Veröffentlichungstag der Anmeldung: 23.01.2008
(73) Patentinhaber: Ziemer Holding AG, 2562 Port (CH)
(72) Erfinder: Rathjen, Christian, 28197 Bremen (DE); Ziemer, Frank, 2562 Port (CH)
(74) Vertreter: Vogel, Dany

(56) Entgegenhaltungen:
- WO-A-20/05070358
- WO-A-20/05092259
- AU-B2- 651 313
- DE-A1- 10 358 927
- DE-A1- 19 950 791
- US-A1- 2004 160 576
- US-A1- 2004 254 568

## Beschreibung

### Technisches Gebiet

Die vorliegende Erfindung betrifft eine ophthalmologische Vorrichtung zur Projektion von Femtosekundenlaserpulsen. Die vorliegende Erfindung betrifft insbesondere eine ophthalmologische Vorrichtung mit einem optischen Lichtprojektionsmodul zur Projektion von abgelenkten Femtosekundenlaserpulsen auf eine definierte Bearbeitungsfläche in einen von der optischen Achse des Lichtprojektionsmoduls ausgedehnten Bildbereich.

### Stand der Technik

Einfache sphärische Linsen fokussieren nur monochromatische Lichtstrahlen im Brennpunkt, die Nahe der optischen Achse verlaufen (Paraxialstrahlen). Strahlen die weiter von der optischen Achse entfernt verlaufen, werden in einem anderen Brennpunkt fokussiert. Dieser geläufigste Abbildungsfehler wird Öffnungsfehler oder auch sphärische Aberration genannt. Soll zur Erzielung kleiner Spotgrößen mit hoher numerischer Apertur fokussiert werden, dann lässt sich dieser Fehler beispielsweise durch asphärische Linsenform kompensieren. Für ausserhalb der optischen Achse liegende Abbildungsorte hilft diese Kompensation nicht. Bei der optischen Abbildung entstehen zudem weitere Aberrationen wie Koma, Bildfeldwölbung, Astigmatismus oder Verzeichnungen. Wird mit mehrfarbigem Licht gearbeitet, kommen noch axial und laterale chromatische Aberrationen hinzu.

Zur Kompensation dieser Fehler werden normalerweise Kombinationen mehrerer sphärischer Linsen mit unterschiedlichen Brechungseigenschaften eingesetzt. Vereinzelt werden dafür auch diffraktiv optische Elemente verwendet. Im allgemeinen steigt der Korrekturaufwand, und damit die Anzahl eingesetzter optischer Elemente (grösstenteils Linsen und Spiegel) und Materialen, mit der numerischen Apertur und der Bildfeldgrösse (Grösse des scharf fokussierten Bildbereichs) steigt. Allerdings steigen mit der numerischen Apertur und der Bildfeldgrösse auch der Durchmesser und das Gewicht der optischen Systeme erheblich. Wenn die Fokalebene zusätzlich verstellt werden soll, steigt der gerätetechnische Aufwand weiter. Dieser Zusammenhang ist insbesondere bei der Konstruktion von Femtolasersystemen von grosser Bedeutung. Zum Beispiel benötigen Femtolasersysteme, die Pulsbreiten von typisch 10fs bis 1000fs (1fs=10⁻¹⁵s) aufweisen, bei einer ophthalmologischen Anwendung numerische Aperturen grösser als 0.2, da ansonsten der Materialabtrag in der Tiefe (z.B. in der Cornea) nicht genau definiert ist und es zum Teil schon auf über der Fokalebene liegenden Strukturen (z.B. im Epithel) zum optischen Durchbruch kommt. Ein weiteres unerwünschtes Phänomen ausserhalb der Fokalebene sind streifenförmige Strukturen im Gewebe entlang der Ausbreitungsrichtung des Laserstrahls (so genannte "streaks"). Selbst bei Systemen mit numerischen Aperturen um die 0.3 gibt es Durchbrüche ober- und unterhalb der Fokalebene. Hoch korrigierte Systeme mit einem Arbeitsgebiet von 10mm mit einer numerischen Apertur von 0.3 benötigen durchaus zehn und mehr Linsen mit einem Durchmesser von um die 100mm. Eine Erhöhung der numerischen Apertur ist praktisch nur noch mit verkleinertem Bildfeld (Bildbereich) möglich. Zusätzlich besteht bei aberrationsbehafteten Abbildungen von Femtosekundenlaserpulsen das Problem, dass durch unterschiedliche Laufzeiten nicht alle Lichtstrahlen zum gleichen Zeitpunkt im Fokus ankommen. Insbesondere bei sehr kurzen Pulsen wird daher die Maximalintensität im Fokus reduziert.

In der Patentanmeldung EP 1486185 wird beispielsweise eine solche ophthalmologische Vorrichtung mit einem manuell einsetzbaren Applikationskopf beschrieben, bei welchem die Vorteile der kleinen Baugrösse und des geringen Gewichts mit dem Nachteil eines eingeschränkten Bildfeldes erkauft werden.

Die Patentanmeldung DE 10358927 beschreibt eine Laservorrichtung zur Materialbearbeitung mittels Laserstrahlung, insbesondere für die refraktive Chirurgie am menschlichen Auge mittels abgelenkter Femtosekundenlaserpulsen. Die Vorrichtung nach DE 10358927 umfasst eine Pulsselektionseinrichtung, welche selektierte Laserpulse so verändert, dass die veränderten Pulse entweder gar nicht mehr in das zu bearbeitende Material gelangen oder zumindest dort keinen optischen Durchbruch mehr erzeugen können. Die Veränderung der Laserpulse umfasst die Beeinflussung von Parametern wie Phase, Amplitude, Polarisation, Strahlrichtung und Feldverteilung über Strahlquerschnitt (Strahlprofil). Insbesondere mittels Wellenfrontveränderung werden die selektierten Laserpulse so defokussiert, dass die Energiedichte nicht mehr zu optischen Durchbrüchen reicht. Die Pulsselektion (und damit die Veränderung) erfolgt insbesondere synchronisiert mit der Ablenkungsgeschwindigkeit.

### Darstellung der Erfindung

Es ist eine Aufgabe der vorliegenden Erfindung eine ophthalmologische Vorrichtung zur Projektion von abgelenkten Femtosekundenlaserpulsen vorzuschlagen, welche nicht die Nachteile des Stands der Technik aufweisen. Es ist insbesondere eine Aufgabe der vorliegenden Erfindung eine ophthalmologische Vorrichtung zur Projektion von abgelenkten Femtosekundenlaserpulsen vorzuschlagen, welche bei gegebener, aus dem Stand der Technik bekannten Projektionsoptik unter Erhaltung der zentralen Abbildungsgüte auch in den Randbereichen der Bearbeitungsfläche (z.B. Fokalebene) ein erweitertes Bildfeld ermöglichen. Es ist insbesondere eine weitere Aufgabe der vorliegenden Erfindung eine ophthalmologische Vorrichtung vorzuschlagen, welche eine derart kleine Bauweise und ein geringes Gewicht aufweist, dass sie für die manuelle Applikation und Positionierung geeignet ist. Weiterhin soll durch die Möglichkeit der Anpassungsfähigkeit der Optik die Verwendung unterschiedlicher Applanationskörper und/oder Wechselobjektive ermöglicht werden.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Die ophthalmologische Vorrichtung umfasst ein optisches Lichtprojektionsmodul zur Projektion von abgelenkten Femtosekundenlaserpulsen in einen von der optischen Achse des Lichtprojektionsmoduls ausgedehnten Bildbereich auf einer definierten Bearbeitungsfläche.

Die oben genannten Ziele werden durch die vorliegende Erfindung insbesondere dadurch erreicht, dass, die ophthalmologische Vorrichtung zudem mit einem steuerbaren optischen Korrekturelement und einem damit verbundenen Steuermodul versehen ist, wobei das steuerbare optische Korrekturelement eingerichtet ist, die Wellenfront der Femtosekundenlaserpulse variabel zu modulieren, und wobei das Steuermodul eingerichtet ist, das Korrekturelement abhängig von einer Ablenkung der Femtosekundenlaserpulse, derart zu steuern, dass die Femtosekundenlaserpulse im Bildbereich fokussiert sind. Vorzugsweise sind das steuerbare optische Korrekturelement und das Steuermodul eingerichtet, die Wellenfront der Femtosekundenlaserpulse sequentiell jeweils für einen innerhalb des Bildbereichs liegenden Bildpunkt zu modulieren, auf welchen Bildpunkt der abgelenkte Laserstrahl projiziert wird. Die sequentielle Korrektur der Wellenfront erfolgt Bildpunkt für Bildpunkt, entsprechend dem Abtastmuster der abgelenkten Laserpulse, und ist bedeutend weniger komplex und aufwendig als eine Korrektur eines gesamten Bildes mit einer Vielzahl von Bildpunkten, die gleichzeitig in den Bildbereich projiziert werden sollen. Die gesteuerte Modulation der Wellenfront ermöglicht die fokussierte Projektion der Femtosekundenlaserpulse auf die Bearbeitungsfläche im gesamten, um die optische Achse des Lichtprojektionsmoduls angeordneten, ausgedehnten Bildbereich. Die Modulation der Wellenfront ermöglicht es, selbst Femtosekundenlaserpulse von gepulsten Laserstrahlen, die entfernt von der optischen Achse des Lichtprojektionsmoduls verlaufen, im Bildbereich fokussiert auf die Bearbeitungsfläche abzubilden. Durch die adaptive und dynamische Modulation der Wellenfront der abgelenkten Femtosekundenlaserpulse können die Femtosekundenlaserpulse bei unveränderter Projektionsoptik des Lichtprojektionsmodul in einem erweiterten Bildbereich auf die Bearbeitungsfläche fokussiert werden, folglich kann bei gleich bleibender kompakter Bauweise eines für die manuelle Applikation geeigneten Lichtprojektionsmoduls das Bildfeld erweitert werden. Durch die korrektive Modulation der Laserpulswellenfronten kann die Abbildungsgüte der Optik des Lichtprojektionsmoduls allgemein verbessert werden. Die gesteuerte Modulation der Wellenfronten ermöglicht die kostengünstigere Bereitstellung eines hochgradig kompensierten optischen Systems mit kleinerer Anzahl optischer Elemente, insgesamt kleinerer Bauweise und geringerem resultierendem Gewicht als optische Systeme des Stands der Technik. Insbesondere ermöglicht das gesteuerte Korrekturmodul die Ausführung einer ophthalmologischen Vorrichtung mit einem kompakten, manuell positionierbaren und für den Patienten nicht bedrohlich gross empfundenen Lichtprojektionsmodul mit einem Bildfeld von mindestens 10mm und einer numerischen Apertur von mindestens 0.3. Kleinere Bildfelder respektive Bildbereiche, z.B. mit einem Durchmesser von 1-2mm, 3-5mm oder 6-9mm, können insbesondere mit einer weiter verkleinerten, noch kompakteren Ausführung erreicht werden. Überdies können bei geregeltem Betrieb Fertigungs- und Montageungenauigkeiten sowie Aberrationen durch leichte optische Aufbauten kompensiert werden.

In einer Ausführungsvariante umfasst die ophthalmologische Vorrichtung ein Detektormodul mit einem Wellenfrontdetektor und/oder einem Lichtstrahlprofildetektor zur Bestimmung eines Wellenfrontverlaufs respektive eines Lichtstrahlprofils der Femtosekundenlaserpulse und das Steuermodul ist eingerichtet, das Korrekturelement abhängig vom bestimmten Wellenfrontverlauf respektive Lichtstrahlprofil zu steuern. Durch die Steuerung des Korrekturelements basierend auf dem erfassten Wellenfrontverlauf respektive Lichtstrahlprofil kann die Wellenfront der Femtosekundenlaserpulse dynamisch moduliert und somit dynamisch für die Fokussierung der Femtosekundenlaserpulse im Bildbereich angepasst werden.

In einer weiteren Ausführungsvariante umfasst die ophthalmologische Vorrichtung optische Elemente zur Erfassung der in den Bildbereich projizierten Femtosekundenlaserpulse, und das Steuermodul ist eingerichtet, das Korrekturelement abhängig von erfassten, in den Bildbereich projizierten Femtosekundenlaserpulsen zu steuern. Die Erfassung der in den Bildbereich projizierten Femtosekundenlaserpulse erlaubt eine möglichst fehlerfreie Rückmeldung und Bestimmung der tatsächlich im Bildbereich erfolgten Projektionsqualität, beispielsweise unter Zuhilfenahme des oben erwähnten Detektormoduls.

In einer Ausführungsvariante umfasst die ophthalmologische Vorrichtung optische Elemente zur Nachbildung eines Referenzstrahlengangs, entsprechend einem für die Projektion der Femtosekundenlaserpulse auf die Bearbeitungsfläche verwendeten Strahlengang, und zur Erfassung von im Referenzstrahlengang projizierten Femtosekundenlaserpulsen. Das Steuermodul ist zudem eingerichtet, das Korrekturelement abhängig von erfassten, im nachgebildeten Strahlengang projizierten Femtosekundenlaserpulsen zu steuern. Die Erfassung der im Referenzstrahlengang projizierten Femtosekundenlaserpulse erlaubt die Rückmeldung und Bestimmung der Projektionsqualität, beispielsweise unter Zuhilfenahme des oben erwähnten Detektormoduls, ohne Eingriff (d.h. Einschub optischer Elemente) in den für die Bearbeitung verwendeten Strahlengang.

In einer Ausführungsvariante umfasst die ophthalmologische Vorrichtung eine Laserquelle zur Erzeugung eines Referenzlaserstrahls, und das Steuermodul ist eingerichtet, das Korrekturelement abhängig vom entsprechend den Femtosekundenlaserpulsen abgelenkten und projizierten Referenzlaserstrahl zu steuern. Der Referenzstrahlengang wird je nach Variante über den für die Bearbeitung verwendeten Strahlengang oder den Referenzstrahlengang geleitet.

In einer Ausführungsvariante umfasst das Steuermodul gespeicherte Steuerdaten mit einer Zuordnung von Steuerwerten zu Ablenkungswerten, und das Steuermodul ist eingerichtet, das Korrekturelement basierend auf den Steuerwerten zu steuern, die den aktuellen Ablenkungswerten zugeordnet sind. Die Steuerung basierend auf definierten Steuerdaten ermöglicht unter anderem eine rückmeldungsfreie Steuerung basierend auf der Kenntnis der Geometrie der ophthalmologischen Vorrichtung.

In einer Ausführungsvariante umfasst die ophthalmologische Vorrichtung optische Elemente, zur Erfassung der in den Bildbereich projizierten Femtosekundenlaserpulse, und ein Kalibrierungsmodul, zum Erzeugen und Abspeichern der Steuerdaten abhängig von erfassten, in den Bildbereich projizierten Femtosekundenlaserpulsen. In einer Variante ist das Kalibrierungsmodul eingerichtet, die Steuerdaten abhängig vom durch das Detektormodul bestimmten Wellenfrontverlauf respektive Lichtstrahlprofil zu erzeugen. Durch die Kalibrierung können die Steuerdaten einerseits nachjustiert und andererseits bei der Applikation an bearbeitungsspezifische Gegebenheiten und Erfordernisse angepasst werden.

In verschiedenen Ausführungsvarianten ist das optische Korrekturelement eingerichtet bei der Modulation der Wellenfront der Femtosekundenlaserpulse die Phasenverteilung, die Amplitudenverteilung, die Polarisationsverteilung und/oder die Ausbreitungsrichtungen im Strahlquerschnitt der Femtosekundenlaserpulse zu verändern.

In einer weiteren Ausführungsvariante umfasst die ophthalmologische Vorrichtung ein Aufnahmeelement zur wechselbaren Aufnahme von mindestens einem Objektivelement des optischen Lichtprojektionsmoduls. Das Aufnahmeelement ermöglicht beispielsweise das Auswechseln des gesamten Lichtprojektionsmoduls oder eines Objektivs oder Objektivelements (z.B. eine oder mehrere Linsen) des Lichtprojektionsmoduls. Insbesondere ermöglicht das Aufnahmeelement die Aufnahme respektive das Auswechseln verschiedener Lichtprojektionsmodule, Objektive respektive Objektivelemente, die für die (fokussierte) Projektion von abgelenkten Femtosekundenlaserpulsen in Bildbereiche unterschiedlicher Grösse und/oder auf verschiedene Bearbeitungsflächen eingerichtet sind, welche Bearbeitungsflächen beispielsweise durch verschiedene Kontaktkörper eben (Applanationsfläche) oder gekrümmt (sphärisch, konvex) definiert (d.h. geformt) sind. Das steuerbare optische Korrekturelement und das Steuermodul sind überdies eingerichtet, die Wellenfront der Femtosekundenlaserpulse so zu modulieren, dass Projektionsfehler, die sich durch das mechanische Auswechseln der Lichtprojektionsmodule, Objektive respektive Objektivelemente ergeben, kompensiert respektive korrigiert werden.

In verschiedenen Ausführungsvarianten umfasst das optische Korrekturelement einen deformierbaren Spiegel, ein räumliches Laufzeit verzögerndes Element, verfahrbare Linsen, verfahrbare Prismen, ein diffraktives optisches Element, ein anamorphotisches Optikmodul, einen photonischen Kristall, einen Linsen-Array, eine räumliche Polarisationsplatte, eine Blende und/oder einen räumlichen Lichtmodulator.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
Figur 1 a zeigt ein Blockdiagramm, welches schematisch eine ophthalmologische Vorrichtung bei der Behandlung eines Auges mittels eines fokussierten gepulsten Laserstrahls darstellt.
Figur 1b zeigt eine Aufsicht einer durch die ophthalmologische Vorrichtung bearbeiteten Bearbeitungsfläche und einem darauf liegenden Bildbereich.
Figuren 2a, 2b, 2c und 2d zeigen jeweils ein Blockdiagramm, welches schematisch eine Ausführungsvariante der ophthalmologischen Vorrichtung bei der Behandlung eines Auges mittels eines fokussierten gepulsten Laserstrahls darstellt.
Figur 3a zeigt zwei gepulste Laserstrahlen, von denen der weiter von der optischen Achse verlaufende Laserstrahl nicht auf eine Bearbeitungsfläche fokussiert abgebildet wird.
Figur 3b zeigt zwei gepulste Laserstrahlen, die beide durch ein "korrigiertes" Lichtprojektionsmodul auf eine Bearbeitungsfläche fokussiert abgebildet werden.

### Wege zur Ausführung der Erfindung

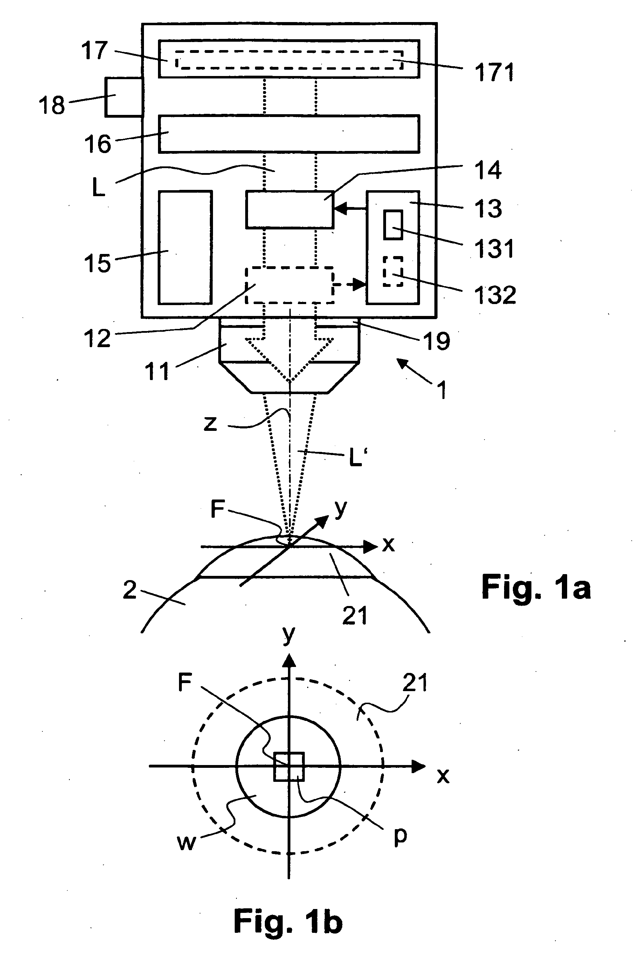

In den Figuren 1 a, 2a, 2b, 2c und 2d bezeichnet das Bezugszeichen 1 eine ophthalmologische Vorrichtung, respektive eine ophthalmologische Vorrichtungsanordnung, mit einer Laserquelle 17 und einem mit der Laserquelle 17 optisch verbundenen optischen Lichtprojektionsmodul 11 zur Erzeugung und fokussierten Projektion eines gepulsten Laserstrahls L' für die punktuelle Gewebeauflösung in einem Fokus F (Brennpunkt) im Innern des Augengewebes 21, beispielsweise in der Hornhaut (Cornea). Die Laserquelle 17 umfasst insbesondere einen Femtosekundenlaser zur Erzeugung von Femtosekundenlaserpulsen, die Pulsbreiten von typisch 10fs bis 1000fs (1fs=10⁻¹⁵s) aufweisen. Die Laserquelle 17 ist in einem separaten oder in einem mit dem Lichtprojektionsmodul 11 gemeinsamen Gehäuse angeordnet. Zum manuellen Halten und Applizieren der ophthalmologischen Vorrichtung 1 respektive des optischen Lichtprojektionsmoduls 11 1 weist die ophthalmologische Vorrichtung 1 einen schematisch dargestellten Handgriff 18 auf. Wie in der Figur 1a zudem schematisch dargestellt ist, umfasst die ophthalmologische Vorrichtung 1 ein Aufnahmeelement 19 zur wechselbaren Aufnahme von verschiedenen optischen Lichtprojektionsmodulen 11 oder zumindest von verschiedenen Objektivelement(en) des Lichtprojektionsmoduls 11. Das Aufnahmeelement 19 ist beispielsweise als Bajonett- oder Schraubverschluss ausgeführt.

Zum besseren Verständnis soll hier angeführt werden, dass die Figuren 1 a, 2a, 2b, 2c und 2d die ophthalmologische Vorrichtung 1 schematisch und vereinfacht darstellen. Zum Beispiel ist in den Figuren nicht präzise wiedergegeben, dass das optische Lichtprojektionsmodul 11 eine hohe numerische Apertur von beispielsweise mindestens 0.3 aufweist, dass die ophthalmologische Vorrichtung 1 mittels eines Saugrings am Auge 2 befestigt ist, und dass die ophthalmologische Vorrichtung 1 einen Kontaktkörper (z.B. einen Applanationskörper) zur kontaktbasierten Verformung (z.B. zur Applanierung) des Auges 2 bei der Applikation der ophthalmologischen Vorrichtung 1 umfasst.

Wie in der Figur 1 a schematisch dargestellt ist, umfasst die ophthalmologische Vorrichtung 1 ein Ablenkungsmodul 16, d.h. ein optisches Abtastmodul oder Scannermodul, welches eingerichtet ist, die von der Laserquelle 17 erzeugten Femtosekundenlaserpulse L1 in mindestens einer Richtung abzulenken und dadurch den Fokus F des gepulsten Laserstrahls L' entsprechend einem Abtastmuster (Scanpattern) mindestens in einer Richtung x, y der (zusammenhängenden oder nicht zusammenhängenden) definierten Bearbeitungsfläche w im Gewebe 21 des Auges 3 zu bewegen. Bei unbewegtem (statischem) Lichtprojektionsmodul 11 bewirkt die Ablenkung der Femtosekundenlaserpulse L1 eine Bewegung des Fokus F des gepulsten Laserstrahls L' im Bildbereich p, der auf der Bearbeitungsfläche w gelegen ist und von der optischen Achse z des Lichtprojektionsmoduls 11 ausdehnt ist (siehe Figur 1b). Der Bildbereich p wird so entsprechend dem Abtastmuster Bildpunkt für Bildpunkt abgetastet, wobei sich einzelne Bildpunkte auch teilweise oder vollständig überlappen können. Im applizierten Zustand der ophthalmologischen Vorrichtung 1 respektive des Lichtprojektionsmoduls 11 verläuft der Bildbereich p im Wesentlichen normal zur optischen Achse z, zumindest im Schnittpunkt mit der optischen Achse z. Es soll hier festgehalten werden, dass die Bearbeitungsfläche w und der Bildbereich p nicht bloss eben sondern auch gekrümmt sein können. Abhängig von der Steuerung des Ablenkungsmoduls 16 weist der Bildbereich p unterschiedliche mögliche Formen auf. Das Ablenkungsmodul 16 ist, beispielsweise zusammen mit der Laserquelle 17, in einem separaten oder in einem mit dem Lichtprojektionsmodul 11 gemeinsamen Gehäuse angeordnet.

Die ophthalmologische Vorrichtung 1 umfasst in einer Variante zudem ein Antriebsmodul 15 um das Lichtprojektionsmodul 11 entlang den Richtungen x und/oder y und/oder einer zu diesen Richtungen Normalen (beispielsweise entlang der optischen Achse z) zu bewegen. Durch das Antriebsmodul 15 kann der Fokus F des gepulsten Laserstrahls L' somit ebenfalls in einer oder mehreren Richtungen bewegt werden. Durch bewegliche Linsen, z.B. innerhalb des Lichtprojektionsmoduls 11, kann der Fokus F des gepulsten Laserstrahls L' auch in der Normalen (z) verstellt werden.

Wie in der Figur 1 a dargestellt ist, umfasst die ophthalmologische Vorrichtung 1 zudem ein steuerbares optisches Korrekturelement 14, das im schematisch dargestellten Strahlengang L zwischen Laserquelle 17 und Austritt des Lichtprojektionsmoduls 11 angeordnet ist. Das optische Korrekturelement 14 ist für die variable Modulation der Wellenfront der Femtosekundenlaserpulse eingerichtet. Durch die steuerbare Modulation der Wellenfront werden die Phasenlaufzeiten und/oder die Ausbreitungsrichtungen über den Strahlquerschnitt der Femtosekundenlaserpulse verändert. Weiterhin können die Polarisation und die Amplitudenverteilung verändert werden. Da die Wellenfront bei unveränderter spektraler Verteilung im allgemeinen von der Amplitudenverteilung, der Polarisationsverteilung und der Phasenverteilung im Strahlquerschnitt abhängig bzw. über diese beeinflusst werden kann, soll im Folgenden stellvertretend für die Amplituden, Polarisations- und Phasenmodulation von einer Wellenfrontmodulation gesprochen werden. Der Begriff Wellenfrontenmodulation umfasst weiterhin die Modulation der Ausbreitungsrichtungen. Das optische Korrekturelement 14 ist beispielsweise als deformierbarer Spiegel, als räumliches Laufzeit verzögerndes Element, mittels verfahrbarer Linsen, mittels verfahrbarer Prismen, und/oder als diffraktives optisches Element ausgeführt. Radial verfahrbare Linsen lassen sich z.B. in Polarkoordinaten-Scannersysteme integrieren, die mit hoher anularer aber vergleichsweise geringer radialer Geschwindigkeit ablenken. Das optische Korrekturelement 14 kann auch mittels eines anamorphotischen Optikmoduls, eines photonischen Kristalls, eines Linsen-Arrays, einer räumlichen Polarisationsplatte, einer Blende und/oder eines räumlichen Lichtmodulators (beispielsweise ein herkömmlicher Spiegelarray oder LCD-Array) ausgeführt werden.

In den Figuren 1 a, 2a, 2b, 2c und 2d bezeichnet das Bezugszeichen 13 ein Steuermodul, das als programmiertes Logikmodul mittels Software und/oder Hardware ausgeführt ist. Das Steuermodul 13 ist zur Übertragung von Steuersignalen mit dem optischen Korrekturelement 14 verbunden. Das Steuermodul 13 ist in einem separaten oder in einem mit dem Lichtprojektionsmodul 11 gemeinsamen Gehäuse angeordnet. Das Steuermodul 13 ist eingerichtet, das optische Korrekturelement 14 so zu steuern, dass die Wellenfront der Femtosekundenlaserpulse so moduliert werden, dass die abgelenkten Femtosekundenlaserpulse des gepulsten Laserstrahls L' jeweils im Bildpunkt fokussiert sind, und zwar für Bildpunkte innerhalb des gesamten Bildbereichs p auf der Bearbeitungsfläche w.

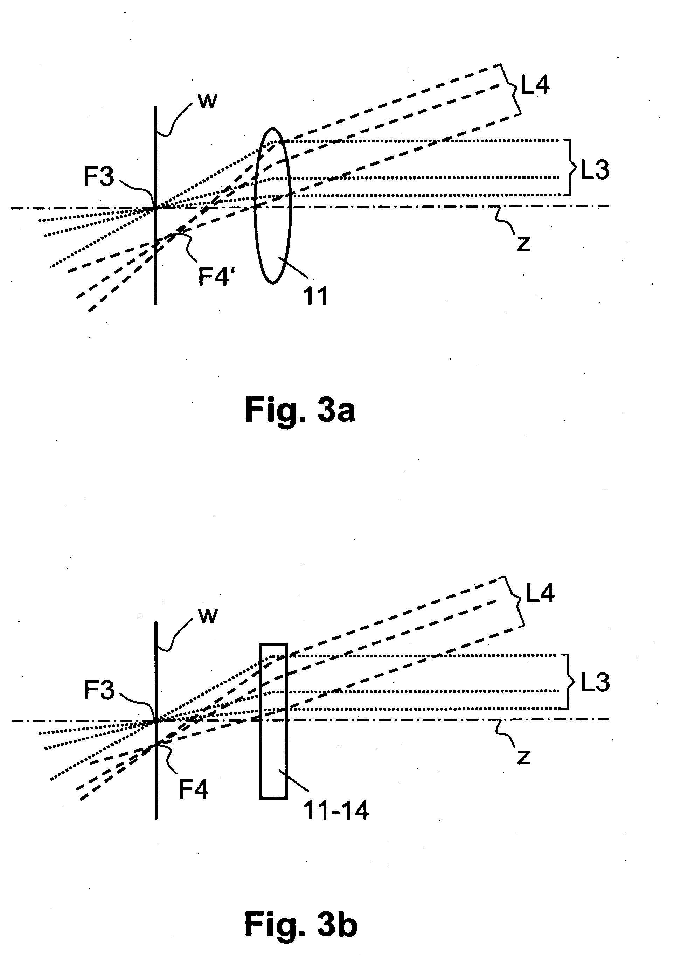

In der Figur 3a ist die Abbildung zweier gepulster Laserstrahlen L3, L4 durch das "unkorrigierte" Lichtprojektionsmodul 11 dargestellt. Der in der Nähe der optischen Achse z verlaufende Laserstrahl L3 wird, wie durch Fokus F3 angedeutet, fokussiert auf die Bearbeitungsfläche w abgebildet. Der weiter von der optischen Achse z abgelenkte Laserstrahl L4 hingegen, wird auf einen ausserhalb der Bearbeitungsfläche w liegenden (unscharfen) Fokusbereich F4' abgebildet. Die Figur 3b illustriert die Abbildung der gepulsten Laserstrahlen L3, L4 durch das "korrigierte" Projektionssystem 11-14, welches das Lichtprojektionsmodul 11 und das optische Korrekturelement 14 umfasst, wobei das Korrekturelement 14 vom Steuermodul 13 so gesteuert wird, dass die Wellenfront der Femtosekundenlaserpulse so moduliert werden, dass sowohl der Laserstrahl L3, im Fokus F3, als auch der Laserstrahl L4, im Fokus F4, fokussiert auf den Bildbereich p der Bearbeitungsfläche w abgebildet werden. Die Steuerung des optischen Korrekturelements 14 erfolgt abhängig von der Ablenkung der von der Laserquelle 17 erzeugten Femtosekundenlaserpulse L1 durch das Ablenkungsmodul 16. Die Ablenkung der Femtosekundenlaserpulse L1 ist beispielsweise durch x/y-Koordinaten im Bildbereich p definiert oder durch einen oder mehrere Ablenkungswinkel. Angaben über die Ablenkung werden beispielsweise vom Ablenkungsmodul 16 an das Steuermodul 13 übertragen oder sind dem Steuermodul 13 bereits aus internen Steuerdaten bekannt. Die Steuerung des optischen Korrekturelements 14 erfolgt zudem in Abhängigkeit von gespeicherten Steuerdaten 131 und/oder Rückmeldungen betreffend die Projektion des gepulsten Laserstrahls L' im Bildbereich p auf der Bearbeitungsfläche w. Die Steuerdaten umfassen für die unterschiedlichen Ablenkungen (Ablenkungswerte) Steuerwerte für die Steuerung des optischen Korrekturelements 14. Die Steuerdaten sind fest definiert, z.B. auf Grund der Berechnung des optischen Systems oder einer Kalibrierung des optischen Systems nach dessen Zusammenbau mittels externer Messinstrumente, oder werden mittels eines optionalen Kalibrierungsmoduls 132 erzeugt, basierend auf Rückmeldungen über die Projektion eines Laserstrahls in den Bildbereich p auf der Bearbeitungsfläche w.

Die Rückmeldung über die Projektion in den Bildbereich p auf der Bearbeitungsfläche w erfolgt mittels optischer Elemente und des optionalen Detektormoduls 12. Insbesondere für die Kalibrierung vor dem operativen Einsatz werden die Femtosekundenlaserpulse des projizierten Laserstrahls L', oder ein hilfsmässiger Referenzlaserstrahl von der optionalen Referenzlaserquelle 171, durch entfernbare respektive bewegliche optische Elemente, beispielsweise Spiegel, halbdurchlässige Spiegel und/oder Linsen, auf dem Niveau der Bearbeitungsfläche w detektiert und optisch dem Detektormodul 12 zugeführt. Für Rückmeldungen während dem operativen Einsatz werden die Femtosekundenlaserpulse, die auf der Bearbeitungsfläche w reflektiert werden, über optische Elemente dem Detektormodul 12 zugeführt beispielsweise mittels reflektierenden und/oder halbdurchlässigen Spiegeln und/oder Linsen. In einer alternativen Ausführungsvariante umfasst die Vorrichtung 1 optische Elemente, beispielsweise Spiegel, halbdurchlässige Spiegel und/oder Linsen, welche einen dem für die Projektion der Femtosekundenlaserpulsen auf die Bearbeitungsfläche w verwendeten Strahlengang entsprechenden Referenzstrahlengang so nachbilden, dass dem Detektormodul 12 Femtosekundenlaserpulse zugeführt werden können, deren Wellenfront und/oder Strahlenprofil denjenigen von Femtosekundenlaserpulsen des Laserstrahls L' im Bildbereich p auf der Bearbeitungsfläche w entsprechen. Der Fachmann wird verstehen, dass Rückmeldungen während dem operativen Einsatz, wie bei der Kalibrierung, in alternativen Ausführungsvarianten auf der Basis eines zusätzlichen hilfsmässigen Referenzlaserstrahls erzeugt werden, der von der optionalen Referenzlaserquelle 171 erzeugt wird.

In verschiedenen Ausführungsvarianten umfasst das Detektormodul 12 einen Wellenfrontdetektor und/oder einen Lichtstrahlprofildetektor zur Bestimmung des Wellenfrontverlaufs respektive des Lichtstrahlprofils der über die optischen Elemente zugeführten Femtosekundenlaserpulse. Der Wellenfrontdetektor ist beispielsweise als Shack-Hartmann-Sensor ausgeführt, beispielsweise nach US 2003/0038921, oder als Interferometer, z.B. als Shearing-Interferometer. Weitere mögliche Ausführungsformen des Wellenfrontdetektors sind in Jos. J. Rozena, Dirk E.M. Van Dyck, Marie-José Tassignon, "Clinical comparison of 6 aberrometers. Part 1: Technical specifications", J Cataract Refract Surg, Volume 31, Juni 2005, Seiten 1114-1127, beschrieben. Der Lichtstrahlprofildetektor umfasst beispielsweise einen CCD-Chip (Charge Coupled Device). Zur Rückmeldung des bestimmten Wellenfrontverlaufs respektive des Lichtstrahlprofils ist das Detektormodul 12 mit dem Steuermodul 13 verbunden. Das Steuermodul 13 ist eingerichtet, basierend auf der Rückmeldung Steuersignale zu erzeugen und an das optische Korrekturmodul 14 zu leiten, um die Wellenfront der Femtosekundenlaserpulse mittels des optischen Korrekturmoduls 14 so zu modulieren, dass sich im Bildbereich p auf der Bearbeitungsfläche w ein gewünschter, definierter Wellenfrontverlauf respektive ein gewünschtes, definiertes Lichtstrahlprofil ergibt und die abgelenkten Femtosekundenlaserpulse auf der Bearbeitungsfläche w über den gesamten Bildbereich p gemäss einem definierten Fokussierungsgrad ausreichend fokussiert sind. Je nach Bearbeitungsverfahren kann es mehrere Gütekriterien für den Fokussierungsgrad geben. Beispielsweise kann die maximale Intensität im Fokus als Fokussierungsgrad definiert werden (wichtig für die Auslösung eines optischen Durchbruchs). Weiterhin kann die Form des Fokus (z.B. kreisförmig für ein definiertes Bearbeitungsgebiet, elliptisch in Abhängigkeit von Abtastmustern, d.h. Scannpattern), dessen Durchmesser (wichtig bei gleichmässigem Spotabstand) oder auch das Intensitätsprofil (z.B. Flattop) als Kriterium für den Fokussierungsgrad dienen. Ist die Fokusform (Spotform) das ausschlaggebende Kriterium, dann kann diese bei ungenügender Intensität an der Laserquelle nachgeregelt werden. Die Nachregelung kann auch in Abhängigkeit von der Fokusposition vorgenommen werden. Die Nachregelung kann beispielsweise erforderlich sein, wenn der Wellfrontmodulator zu schwankenden Intensitäten führt.

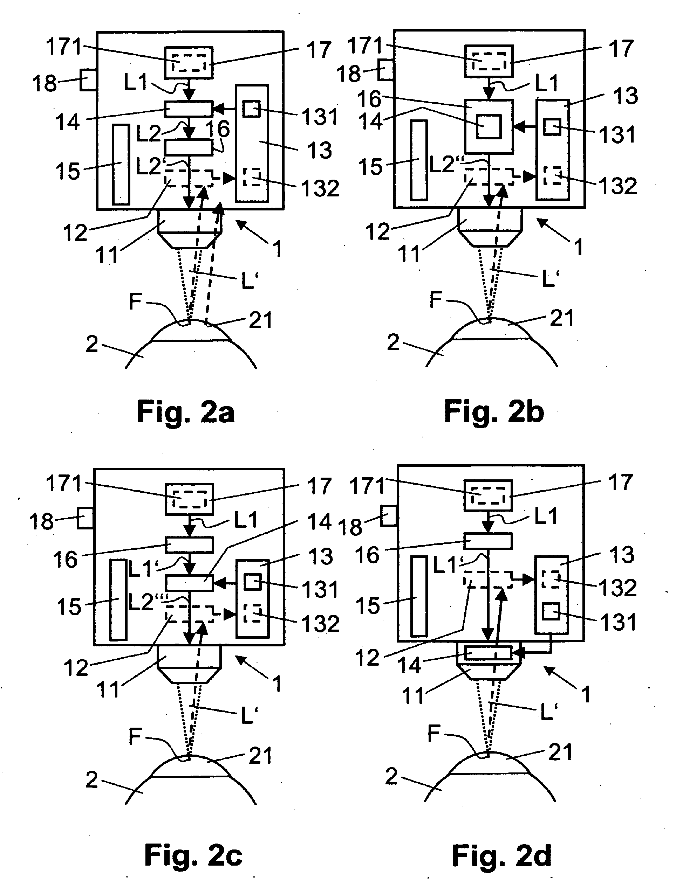

In der Ausführungsvariante nach Figur 2a ist das steuerbare optische Korrekturelement 14 in den Strahlengang zwischen der Laserquelle 17 und dem Ablenkungsmodul 16 eingefügt. Die von der Laserquelle 17 erzeugten Femtosekundenlaserpulse L1 werden dem Korrekturelement 14 zugeführt, welches die Wellenfront der Femtosekundenlaserpulse L1 entsprechend den Steuersignalen des Steuermoduls 13 jeweils für den Bildpunkt moduliert, auf welchen sie im Bildbereich p abgelenkt und projiziert werden. Die vom Korrekturelement 14 erzeugten Femtosekundenlaserpulse L2 mit modulierter Wellenfront werden dem Ablenkungsmodul 16 zugeführt. Das Ablenkungsmodul 16 lenkt die Femtosekundenlaserpulse L2 in mindestens eine Abtastrichtung ab. Die vom Ablenkungsmodul 16 abgelenkten Femtosekundenlaserpulse L2' werden dem Lichtprojektionsmodul 11 zur fokussierten Projektion zugeführt.

In der Ausführungsvariante nach Figur 2b ist das steuerbare optische Korrekturelement 14 in das Ablenkungsmodul 16 integriert. Die von der Laserquelle 17 erzeugten Femtosekundenlaserpulse L1 werden dem Ablenkungsmodul 16 zugeführt. Im internen Strahlengang des Ablenkungsmoduls 16 moduliert das Korrekturelement 14 die Wellenfront der Femtosekundenlaserpulse L1 entsprechend den Steuersignalen des Steuermoduls 13 jeweils für den Bildpunkt, auf welchen sie im Bildbereich p abgelenkt und projiziert werden. Die vom Korrekturelement 14 modulierten und vom Ablenkungsmodul 16 in mindestens einer Abtastrichtung abgelenkten Femtosekundenlaserpulse L2" mit modulierter Wellenfront werden dem Lichtprojektionsmodul 11 zur fokussierten Projektion zugeführt.

In der Ausführungsvariante nach Figur 2c ist das steuerbare optische Korrekturelement 14 in den Strahlengang zwischen dem Ablenkungsmodul 16 und dem Lichtprojektionsmodul 11 eingefügt. Die von der Laserquelle 17 erzeugten und vom Ablenkungsmodul 16 abgelenkten Femtosekundenlaserpulse L1' werden dem Korrekturelement 14 zugeführt. Das Korrekturelement 14 moduliert die Wellenfront der abgelenkten Femtosekundenlaserpulse L1' entsprechend den Steuersignalen des Steuermoduls 13 jeweils für den Bildpunkt, auf welchen sie im Bildbereich p abgelenkt und projiziert werden. Die vom Ablenkungsmodul 16 in mindestens einer Abtastrichtung abgelenkten und vom Korrekturelement 14 modulierten Femtosekundenlaserpulse L2"' mit modulierter Wellenfront werden dem Lichtprojektionsmodul 11 zur fokussierten Projektion zugeführt. Im Falle eines Ablenkspiegels im Ablenkungsmodul 16 kann dieser gleichzeitig auch verformbar oder intensitätsmodulierend ausgeführt werden und somit auch als optisches Korrekturelement dienen. Weiterhin kann z.B. ein deformierbarer Spiegel zusätzlich zu der auf der Wellenfrontmodulierung basierenden Korrektur auch zur Verstellung (Positionierung) des Fokus eingesetzt werden.

In der Ausführungsvariante nach Figur 2d ist das steuerbare optische Korrekturelement 14 in das Lichtprojektionsmodul 11 integriert. Die von der Laserquelle 17 erzeugten Femtosekundenlaserpulse L1 werden dem Ablenkungsmodul 16 zugeführt, welche die Femtosekundenlaserpulse L1 in mindestens eine Abtastrichtung ablenken. Die abgelenkten Femtosekundenlaserpulse L1' werden dem Korrekturelement 14 im Lichtprojektionsmodul 11 zugeführt, welches die Wellenfront der abgelenkten Femtosekundenlaserpulse L1' jeweils für den Bildpunkt moduliert, auf welchen sie im Bildbereich p abgelenkt und projiziert werden. Die vom Korrekturelement 14 erzeugten Femtosekundenlaserpulse mit modulierter Wellenfront werden vom Lichtprojektionsmodul 11 fokussiert projiziert.

## Patentansprüche

1. Ophthalmologische Vorrichtung (1), umfassend:
ein optisches Lichtprojektionsmodul (11) zur Projektion von abgelenkten Femtosekundenlaserpulsen in einen von der optischen Achse (z) des Lichtprojektionsmoduls (11) ausgedehnten Bildbereich (p) auf einer definierten Bearbeitungsfläche (w), und
ein steuerbares optisches Korrekturelement (14) zur variablen Modulation der Wellenfront der Femtosekundenlaserpulse,
**gekennzeichnet durch**,
ein mit dem Korrekturelement (14) verbundenes Steuermodul (13) zur Steuerung des Korrekturelements (14) abhängig von einer Ablenkung der Femtosekundenlaserpulse, derart dass die Femtosekundenlaserpulse **durch** die adaptive und dynamische Modulation der Wellenfront im ausgedehnten Bildbereich (p) auf der definierten Bearbeitungsfläche (w) fokussiert werden.

2. Vorrichtung (1) nach Anspruch 1, **dadurch gekennzeichnet, dass** das steuerbare optische Korrekturelement (14) und das Steuermodul (13) eingerichtet sind, die Wellenfront der Femtosekundenlaserpulse sequentiell jeweils für einen innerhalb des Bildbereichs (p) liegenden Bildpunkt zu modulieren.

3. Vorrichtung (1) nach einem der Ansprüche 1 oder 2, **gekennzeichnet durch** ein Detektormodul (12) umfassend mindestens einen aus Wellenfrontdetektor und Lichtstrahlprofildetektor zur Bestimmung eines Wellenfrontverlaufs respektive Lichtstrahlprofils der Laserpulse, und **dadurch**, dass das Steuermodul (13) eingerichtet ist, das Korrekturelement (14) abhängig vom bestimmten Wellenfrontverlauf respektive Lichtstrahlprofil zu steuern.

4. Vorrichtung (1) nach einem der Ansprüche 1 bis 3, **gekennzeichnet durch** optische Elemente zur Erfassung der in den Bildbereich (p) projizierten Femtosekundenlaserpulse, und **dadurch**, dass das Steuermodul (13) eingerichtet ist, das Korrekturelement (14) abhängig von erfassten, in den Bildbereich (p) projizierten Femtosekundenlaserpulsen zu steuern.

5. Vorrichtung (1) nach einem der Ansprüche 1 bis 3, **gekennzeichnet durch** optische Elemente zur Nachbildung eines Referenzstrahlengangs, entsprechend einem für die Projektion der Femtosekundenlaserpulsen auf die Bearbeitungsfläche (w) verwendeten Strahlengang, und zur Erfassung von im Referenzstrahlengang projizierten Femtosekundenlaserpulsen, und **dadurch**, dass das Steuermodul (13) eingerichtet ist, das Korrekturelement (14) abhängig von erfassten, im nachgebildeten Strahlengang projizierten Femtosekundenlaserpulsen zu steuern.

6. Vorrichtung (1) nach einem der Ansprüche 1 bis 5, **gekennzeichnet durch** eine Laserquelle (171) zur Erzeugung eines Referenzlaserstrahls, und **dadurch**, dass das Steuermodul (13) eingerichtet ist, das Korrekturelement (14) abhängig vom entsprechend den Femtosekundenlaserpulsen abgelenkten und projizierten Referenzlaserstrahl zu steuern.

7. Vorrichtung (1) nach Anspruch 1, **dadurch gekennzeichnet, dass** das Steuermodul (13) gespeicherte Steuerdaten (131) mit einer Zuordnung von Steuerwerten zu Ablenkungswerten umfasst, und dass das Steuermodul (13) eingerichtet ist, das Korrekturelement (14) basierend auf den Steuerwerten (131) zu steuern, die aktuellen Ablenkungswerten zugeordnet sind.

8. Vorrichtung (1) nach Anspruch 7, **gekennzeichnet durch** optische Elemente zur Erfassung der in den Bildbereich (p) projizierten Femtosekundenlaserpulse, und **durch** ein Kalibrierungsmodul (132) zum Erzeugen und Abspeichern der Steuerdaten (131) abhängig von erfassten, in den Bildbereich (p) projizierten Femtosekundenlaserpulsen.

9. Vorrichtung (1) nach einem der Ansprüche 7 oder 8, **gekennzeichnet durch** ein Detektormodul (12) umfassend mindestens einen aus Wellenfrontdetektor und Lichtstrahlprofildetektor zur Bestimmung eines Wellenfrontverlaufs respektive Lichtstrahlprofils der Femtosekundenlaserpulse, und **dadurch**, dass das Kalibrierungsmodul (12) eingerichtet ist, die Steuerdaten (131) abhängig vom bestimmten Wellenfrontverlauf respektive Lichtstrahlprofil zu erzeugen.

10. Vorrichtung (1) nach einem der Ansprüche 1 bis 9, **dadurch gekennzeichnet, dass** das optische Korrekturelement (14) eingerichtet ist bei der Modulation der Wellenfront der Femtosekundenlaserpulse mindestens eines aus Phasenverteilung, Amplitudenverteilung, Polarisationsverteilung und Ausbreitungsrichtungen im Strahlquerschnitt der Femtosekundenlaserpulse zu verändern.

11. Vorrichtung (1) nach einem der Ansprüche 1 bis 10, **dadurch gekennzeichnet, dass** das optische Korrekturelement (14) mindestens eines aus deformierbarer Spiegel, räumliches Laufzeit verzögerndes Element, verfahrbare Linsen, verfahrbare Prismen, diffraktives optisches Element, anamorphotisches Optikmodul, photonischer Kristall, Linsen-Array, räumliche Polarisationsplatte, Blende und räumlicher Lichtmodulator umfasst.

12. Vorrichtung (1) nach einem der Ansprüche 1 bis 11, **gekennzeichnet durch** einen Handgriff (18) zum manuellen Halten und Applizieren der ophthalmologischen Vorrichtung (1), und **dadurch**, dass das optische Lichtprojektionsmodul (11) eine numerische Apertur von mindestens 0.3 aufweist, und dass der Bildbereich (p) auf der Bearbeitungsfläche (w) einen Durchmesser von mindestens 10mm aufweist.

13. Vorrichtung (1) nach einem der Ansprüche 1 bis 12, **gekennzeichnet durch** ein Aufnahmeelement (19) zur wechselbaren Aufnahme von mindestens einem Objektivelement des optischen Lichtprojektionsmoduls (11).

## Claims

1. Ophthalmological device (1) comprising:
an optical light projection module (11) for projecting deflected femtosecond laser pulses into an image region (p), extending from the optical axis (z) of the light projection module (11), on a defined treatment surface (w), and
- a controllable optical correction element (14) for variable modulation of the wavefront of the femtosecond laser pulses,
**characterized by**
a control module (13), connected to the correction element (14), for controlling the correction element (14) as a function of a deflection of the femtosecond laser pulses in such a way that the femtosecond laser pulses are focused by the adaptive and dynamic modulation of the wavefront in the extended image region (p) on the defined treatment surface (w).

2. Device (1) according to Claim 1, **characterized in that** the controllable optical correction element (14) and the control module (13) are set to modulate the wavefront of the femtosecond laser pulses sequentially in each case for a pixel lying inside the image region (p).

3. Device (1) according to either of Claims 1 and 2, **characterized by** a detector module (12) comprising at least one of wavefront detector and light beam profile detector for determining a wavefront profile or light beam profile of the laser pulses, and in that the control module (13) is set up to control the correction element (14) as a function of the specific wavefront profile or light beam profile.

4. Device (1) according to one of Claims 1 to 3, **characterized by** optical elements for acquiring the femtosecond laser pulses projected into the image region (p) and in that the control module (13) is set up to control the correction element (14) as a function of acquired femtosecond laser pulses projected into the image region (p).

5. Device (1) according to one of Claims 1 to 3,
**characterized by** optical elements for simulating a reference beam path corresponding to a beam path used for projection of the femtosecond laser pulses onto the treatment surface (w) and for acquiring femtosecond laser pulses projected in the reference beam path, and in that the control module (13) is set up to control the correction element (14) as a function of acquired femtosecond laser pulses projected in the simulated beam path.

6. Device (1) according to one of Claims 1 to 5, **characterized by** a laser source (171) for generating a reference laser beam, and in that the control module (13) is set up to control the correction element (14) as a function of the reference laser beam deflected and projected in accordance with the femtosecond laser pulses.

7. Device (1) according to Claim 1, **characterized in that** the control module (13) includes stored control data (131) with an assignment of control values to deflection values, and **in that** the control module (13) is set up to control the correction element (14) on the basis of the control values (131) which are assigned to current deflection values.

8. Device (1) according to Claim 7, **characterized by** optical elements for acquiring the femtosecond laser pulses projected into the image region (p) and by a calibration module (132) for generating and storing control data (131) as a function of acquired femtosecond laser pulses projected into the image region (p).

9. Device (1) according to either of Claims 7 or 8, **characterized by** a detector module (12) comprising at least one of wavefront detector and light beam profile detector for determining a wavefront profile or light beam profile of the femtosecond laser pulses, and in that the calibration module (132) is set up to generate control data (131) as a function of the determined wave front profile or light beam profile.

10. Device (1) according to one of Claims 1 to 9, **characterized in that** the optical correction element (14) is set up to vary at least one of phase distribution, amplitude distribution, polarization distribution and propagation directions in the beam cross section of the femtosecond laser pulses during the modulation of the wavefront of the femtosecond laser pulses.

11. Device (1) according to one of Claims 1 to 10, **characterized in that** the optical correction element (14) includes at least one of the following: a deformable mirror, a spatial propagation time delaying element, movable lenses, movable prisms, a diffractive optical element, an anamorphic optical module, a photonic crystal, a lens array, spatial polarization plate, a diaphragm and a spatial light modulator.

12. Device (1) according to one of Claims 1 to 11, **characterized by** a handle (18) for manually holding and applying the ophthalmological device (1), and in that the optical light projection module (11) has a numerical aperture of at least 0.3, and in that the image region (p) on the treatment surface (w) has a diameter of at least 10 mm.

13. Device (1) according to one of Claims 1 to 12, **characterized by** a holding element (19) for changeably holding at least one objective element of the optical light projection module (11).

## Revendications

1. Dispositif ophtalmologique (1) comprenant :
un module optique de projection de lumière (11) pour projeter des impulsions laser femtoseconde déviées dans une zone d'image (p) expansée à partir de l'axe optique (z) du module de projection de lumière (11) sur une surface de traitement (w) définie, et
un élément correcteur optique commandable (14) pour réaliser la modulation variable du front d'onde de l'impulsion laser femtoseconde,
**caractérisé par**
un module de commande (13) relié avec l'élément correcteur (14) pour commander l'élément correcteur (14) en fonction d'une déviation de l'impulsion laser femtoseconde de manière à réaliser la focalisation sur la surface de traitement (w) définie par la modulation adaptative et dynamique du front d'onde dans la zone d'image expansée (p).

2. Dispositif (1) selon la revendication 1,
**caractérisé en ce que** l'élément correcteur optique commandable (14) et le module de commande (13) sont configurés pour moduler séquentiellement le front d'onde de l'impulsion laser femtoseconde à chaque fois pour un pixel qui se trouve à l'intérieur de la zone d'image (p).

3. Dispositif (1) selon la revendication 1 ou 2, **caractérisé par** un module détecteur (12) comprenant au moins un détecteur de front d'onde et un détecteur de profil de rayon lumineux pour déterminer un tracé de front d'onde ou un profil de rayon lumineux de l'impulsion laser, et en ce que le module de commande (13) est configuré pour commander l'élément correcteur (14) en fonction du tracé de front d'onde ou du profil de rayon lumineux déterminé.

4. Dispositif (1) selon l'une des revendications 1 à 3, **caractérisé par** des éléments optiques destinés à détecter l'impulsion laser femtoseconde projetée dans la zone d'image (p) et en ce que le module de commande (13) est configuré pour commander l'élément correcteur (14) en fonction des impulsions laser femtoseconde détectées et projetées dans la zone d'image (p).

5. Dispositif (1) selon l'une des revendications 1 à 3, **caractérisé par** des éléments optiques destinés à simuler un trajet de rayon de référence correspondant à un trajet de rayon utilisé pour la projection des impulsions laser femtoseconde sur la surface de traitement (w) et à détecter les impulsions laser femtoseconde projetées dans le trajet de rayon de référence, et en ce que le module de commande (13) est configuré pour commander l'élément correcteur (14) en fonction des impulsions laser femtoseconde détectées et projetées dans le trajet de rayon simulé.

6. Dispositif (1) selon l'une des revendications 1 à 5, **caractérisé par** une source laser (171) destinée à générer un trajet de rayon de référence et en ce que le module de commande (13) est configuré pour commander l'élément correcteur (14) en fonction du trajet de rayon de référence dévié et projeté conformément aux impulsions laser femtoseconde.

7. Dispositif (1) selon la revendication 1,
**caractérisé en ce que** le module de commande (13) comprend des données de commande (131) mémorisées avec une association entre les valeurs de commande et les valeurs de déviation et **en ce que** le module de commande (13) est configuré pour commander l'élément correcteur (14) en se basant sur les valeurs de commande (131) qui sont associées aux valeurs de déviation actuelles.

8. Dispositif (1) selon la revendication 7,
**caractérisé par** des éléments optiques pour détecter l'impulsion laser femtoseconde projetée dans la zone d'image (p) et par un module de calibrage (132) destiné à générer et à mémoriser les données de commande (131) en fonction des impulsions laser femtoseconde détectées et projetées dans la zone d'image (p).

9. Dispositif (1) selon l'une des revendications 7 ou 8, **caractérisé par** un module détecteur (12) comprenant au moins un détecteur de front d'onde et un détecteur de profil de rayon lumineux pour déterminer un tracé de front d'onde ou un profil de rayon lumineux de l'impulsion laser femtoseconde, et en ce que le module de calibrage (132) est configuré pour générer les données de commande (131) en fonction du front d'onde ou du profil de rayon lumineux déterminé.

10. Dispositif (1) selon l'une des revendications 1 à 9, **caractérisé en ce que** l'élément de correction optique (14), lors de la modulation du front d'onde de l'impulsion laser femtoseconde, est configuré pour modifier au moins l'un des éléments suivantes : distribution de phase, distribution d'amplitude, distribution de polarisation et sens de propagation dans la section transversale du rayon de l'impulsion laser femtoseconde.

11. Dispositif (1) selon l'une des revendications 1 à 10, **caractérisé en ce que** l'élément correcteur optique (14) comprend au moins l'un des éléments suivants : miroir déformable, élément retardant le temps de propagation spatial, lentilles mobiles, prismes mobiles, élément optique diffractif, module optique anamorphique, cristal photonique, réseau de lentilles, plaque de polarisation dans l'espace, obturateur et modulateur de lumière dans l'espace.

12. Dispositif (1) selon l'une des revendications 1 à 11, **caractérisé par** une poignée (18) pour tenir manuellement et appliquer le dispositif ophtalmologique (1), et en ce que le module optique de projection de lumière (11) présente une ouverture numérique d'au moins 0,3, et en ce que la zone d'image (p) sur la surface de traitement (w) présente un diamètre d'au moins 10 mm.

13. Dispositif (1) selon l'une des revendications 1 à 12, **caractérisé par** un élément de logement (19) pour un logement avec possibilité de remplacement d'un élément d'objectif du module optique de projection de lumière (11).
